# EUROPEAN PATENT SPECIFICATION

(11) **EP 0 299 745 B1**
(45) Date of publication and mention of the grant of the patent: **31.05.1995**
(21) Application number: 88306419.8
(22) Date of filing: 13.07.1988
(51) Int. Cl.: A61K 31/70, C07H 21/00

(54) **Double-stranded RNA correction of RNase L deficiency**
Behandlung der RNase L-Defizienz mit ds RNS
Correction d'une insuffisance de RNAse L par un ds ARN

(30) Priority: 17.07.1987 US 74649
(43) Date of publication of application: 18.01.1989
(60) Divisional application: 92203073.9
(73) Proprietor: HEM PHARMACEUTICALS CORP., Rockville, MD 20852 (US)
(72) Inventor: Carter, William A., Birchrunville, PA 19103 (US)
(74) Representative: Votier, Sidney David

(56) References cited:
- EP-A- 0 113 162
- EP-A- 0 213 921
- THE LANCET, 06 June 1987; W. CARTER et al., p. 1286#
- CHEMICAL ABSTRACTS, vol. 104, no. 19, 12 May 1986, Columbus, OH (US); H. HUBBEL et al., no. 166709#
- CHEMICAL ABSTRACTS, vol. 104, no. 16, 21 April 1986, Columbus, OH (US); SUHADOLNIK et al., no. 141833#
- BIOLOGICAL ABSTRACTS; H. HUBBEL et al., no. 33061246#
- PROCEEDINGS OF THE AMERICAN ASSOCIATION OF CANCER RESEARCH, vol. 26, March 1985; ROSENBLUM et al., p. 280, no. 1105#
- NUCLEIC ACIDS RESEARCH, vol. 9, no. 7, 1981; D.H. WRESCHNER et al., pp. 1571-1580#
- BIOCHEM. & BIOPHYS. RESEARCH COMM., vol. 128, 1985; pp. 695-698#

## Description

Historically, various human lymphokines (interferons, interleukins, tumor necrosis factors, etc.) have proven to have modest clinical utility thus far in most human neoplasias and chronic viral infections. Principally, it may be because these lymphokines only enhance the "upstream" part of an elaborate biochemical cascade; for example, they can operate at the cell surface receptor level for polypeptides or in the early 2′-5′ A synthetase enzyme induction mechanism.

In contrast, I describe herein a novel defect in a more critical and terminal component of the lymphokine action/natural defense pathway, namely an abnormality in RNA-ase L, which abnormality or defect if uncorrected can lead to uncontrolled tumor cell growth as well as proliferation of various viruses in host cells. I describe now both the new biochemical defect in RNA-ase L modulation as well as a wholly new process for stabily correcting said defects. The stable correction of these defects by dsRNAs in general and mismatched dsRNAs in particular is associated with dramatic clinical changes including renewal of activity against various tumors by the body as well as the reappearance of broad-spectrum capacity to inhibit various viral and fungal pathogens which often afflict both cancer patients and other individuals in various "high risk" groups.

### BACKGROUND OF THE INVENTION

Lymphokines have limited efficacy in the control of either human tumors (Carter et al, J. Biol. Res. Mod., Vol. 4:613, 1985), or human viruses (see references cited in Montefiori and Mitchell, Proc. Natl. Acad. Sci. U.S., Vol. 84, page 2985, 1987). In an attempt to enhance their activities, scientists have sought to combine these natural polypeptides with various other compounds.

**2-5A Synthetase:** For example, Rosenblum and Gutterman (Proc. Am. Assoc. Cancer Research, Vol 26, March 1985, page 280, Abstract No. 1105) combined interferon (IFN) with dsRNA. They reported an "impressive synergistic antiproliferative activity" on human melanoma in cell culture by sub-effective concentrations of dsRNA and IFN given together. However, they found that the synergy was "limited to antiproliferative IFN antiviral activity". They observed increases in 2′-5′ oligo A synthetase by the combination approach, but recognized that "the synergistic antiproliferative properties of this combination may not be mediated through an effect on 2,5 A." In contrast, elevated 2′-5′ A synthetase following IFN therapy with IFN alone in treatment of chronic hepatitis B (CHB) virus infection (Furuta et al, J. Interferon Res., Vol 7, page 111). In general, however, attempts to use 2′-5′ A synthetase activity in peripheral blood lymphocytes (PBL) as a marker to estimate to what degree various tumors or viruses are sensitive to lymphokines has been some what disappointing.

I believe this is the case because 2′5′A synthetase is only one of various enzymes positioned early in the biochemical cascade induced by lymphokines and especially because 2′5′A synthetase can primarily exert antiviral or antitumor action via activating a more distal enzyme in the pathway(s) termed RNA-ase L. A recent description of purified 2′5′ oligo A synthetase showed that it was highly unstable in the presence of dsRNA (Rovnak and Ranu, J. Interferon Res., Vol 7, page 231, 1987).

The use of mismatched dsRNA in the treatment of cancer or immunological or viral disease is described in EP-A- 0 213 921

I have now elucidated a new role for dsRNA in terms of correcting various aberrancies in RNA-ase L which corrections I can also show are of therapeutic importance in successful treatment of cancer, immunological and viral diseases.

**RNA-ase L:** RNA-ase L, when properly activated, is associated with the specific cleavage of viral RNA and presumably aberrant cellular RNAs associated with the malignant cell phenotype (uncontrolled proliferation). In a recent publication (Lancet, June 6, 1987, Vol. 1, p. 1286), I describe a different defect in RNA-ase L secondary to the elaboration of a HIV (Human Immunodeficiency Virus) inhibitor of RNA-ase L. I devised a successful therapeutic regimen which consisted of dsRNA dosage schedules which permit, by either direct or indirect mechanisms of action, the displacement of the HIV inhibitor from RNA-ase L resulting in improvement of the patient's clinical status and reduction of HIV load.

### BRIEF DESCRIPTION OF THE DRAWINGS

FIGURES 1-3 are photographs of polyacrylamide gel electrophoresis plates showing the indicated number of tracks and bands or zones along each track; and
FIGURE 4 is a three-part graph showing three different patients and comparing the white blood cell count (the line connected with the O's), with 2-5 A synthetase quantity (the line connected with the deltas) compared to the days of therapy.

### DETAILED DESCRIPTION OF THE INVENTION

Herein, I describe a new and completely distinct additional anomaly in RNA-ase L function. Again, the anomaly is associated with severe clinical disease, but judicious use of dsRNAs permits correction of the RNA-ase L anomaly with concurrent clinical improvement.

The invention includes diagnostic procedures for assessing defects and biochemical aberrances in RNA-ase L and therapeutic methods of treatment for correcting such defects when encountered by administering a dsRNA, preferably a mismatched dsRNA, optionally prior to or concurrently with the administration of a lymphokine, such as an interferon or an interleukin. Procedures for determining the presence of RNA-ase L deficiency-characteristic cleavage products in a sample of the patient's blood, in particular the patient's mononuclear cells, and using the presence and/or time of formation of such cleavage products as a marker to initiate therapy with dsRNAs are also described.

Therapeutic methods of treating cancers, particularly those having tumor cells resistant to treatment with an interferon alone, and viral conditions including diseases caused by members of the retrovirus family are included in this invention.

In the previously-cited Lancet article, Figure 5, RNA-ase L samples from HIV infected individuals failed to show any detectable bioactivity. In particular, the "SCP" zones, i.e., Specific Cleavage Product zones, were devoid of RNA fragments which demonstrated that the RNA-ase L from these patients had been effectively inactivated by inhibitor. In contrast, healthy normal subjects showed in vivo activated RNA-ase L quite readily. Thus, aberrant RNA-ase L in human tumors is associated with novel cleavage products (NCP).

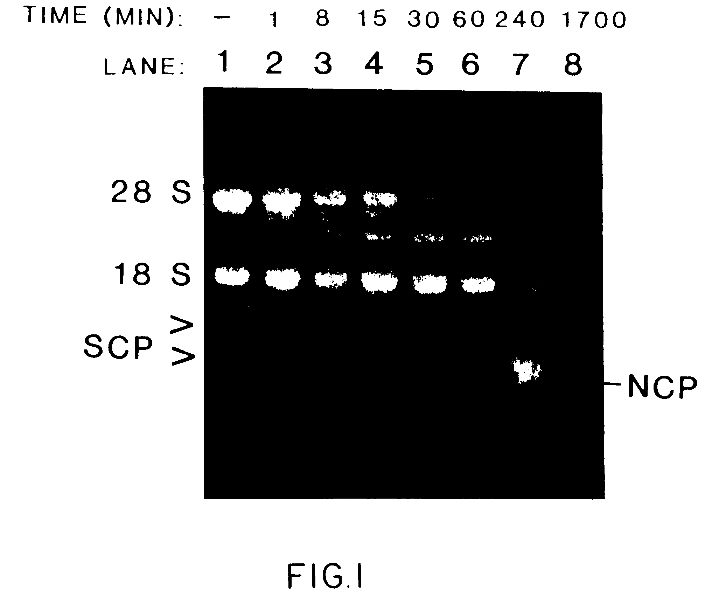

Novel cleavage products (NCP) are different from specific cleavage products (SCP), and identify human cells without normal mechanisms to control cell growth and virus proliferation. Figure 1, a photograph of a polyacrylamide gel electrophoresis (PAGE) plate, demonstrates the kinetics of RNA-ase L activity obtained from peripheral lymphocytes of a normal individual similar to Lane 2, Figure 5 of my previous article (Lancet, cited above). As previously described, the analytic method consisted of measuring activated RNA-ase L in cytoplasmic extract of mononuclear cells by determining the ability to generate specific cleavage products when incubated with a source of 28S and 18S RNA. The procedures employed follow those described by K. Kariko and J. Ludwig, Biochem. Biophysics. Research Communication, Vol. 128, p. 695, 1985 and Wreschner et al, Nucleic Acid Research, Vol. 9, p. 1571, 1981. Ribosomal RNA was obtained from HL929 cells, which genetically lack RNA-ase L. These LH929 cells are deposited under the terms of the Budapest Treaty at the American Type Culture Collection, Rockville, Maryland, USA as ATCC accession no. CRL9659. By observing over an extended time period of 1700 minutes (about 26 hours), I found that the RNA-ase L from normal individuals was eventually able to form NCP (Lane 8 of Figure 1). Note that the earliest point of detection of NCP in a normal individual is after many hours of incubation.

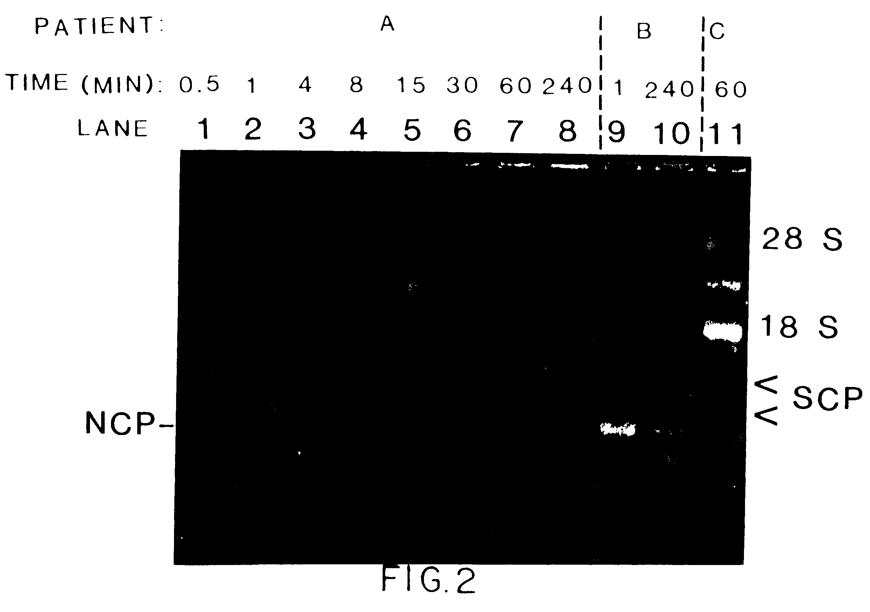

However, NCP are seen within minutes of RNA-ase L incubation when lymphocytes from patients with leukemia are used. This is clearly shown in Figure 2, when cells from 3 leukemic patients (chronic myelogenous leukemia of CML patients A and B are compared with comparable cells from a normal individual, patient C). Notice that in the leukemia patients (Patient A and B, Figure 2) SCP are never seen and NCP are seen after incubations as brief as 1 to 4 minutes. In striking contrast, in a normal individual (volunteer designed C), only SCP -- and no NCP whatsoever -- is seen in kinetic samples taken up to 60 minutes.

Accordingly, I have observed a previously undetected biochemical anomaly in which a key enzyme (RNA-ase L) associated with the body defense mechanisms against cancer, immunological and viral diseases is operating in an accelerated and apparently uncontrolled manner. In separate experiments, I compared the relative abilities of these two different cells (cells with abnormal RNA-ase L) to withstand viral challenge. I observed that the titers (yields) of progeny viruses were significantly higher in those cells with the abnormal RNA-ase L activity which generated NCP so rapidly.

I have discovered and hereby disclose a procedure in which double-stranded RNAs, especially mismatched dsRNAs, restore normalcy of RNA-ase L kinetics and degradation products and that the rate of restoration of normalcy by double-stranded RNA can be accelerated by prior exposure to lymphokines.

By "mismatched dsRNA" are meant those in which hydrogen bonding (base stacking) between the counterpart strands is relatively intact, i.e., is interrupted on average less than one base pair in every 29 consecutive base residues. The term "mismatched dsRNA" should be understood accordingly. The dsRNA may be a complex of a polyinosinate and a polycytidylate containing a proportion of uracil bases or guanidine bases, e.g., from 1 in 5 to 1 in 30 such bases (poly I·poly (C₄-C₂₉ x > U or G). Alternatively, appropriate oligonucleotides (small nucleotide fragments) may be complexed with appropriate complementary polynucelotides or oligonucleotides under certain circumstances.

The dsRNA may be poly I·poly C,U in which the ratio of C to U is about 13 to 1 and the sedimentation coefficients of poly I and poly C,U are both less than 9 and within 2 units of each other, and both preferably about 6.5 to 7.5.

The dsRNA may be of the general formula rIₙ.r(C₁₁₋₁₄,U)ₙ and specifically, rIₙ.r(C₁₂,U)ₙ. Other suitable examples of dsRNA are discussed below.

The mismatched dsRNAs preferred for use in the present invention are based on copolynucelotides selected from poly (Cₙ,U) and poly (Cₙ,G) in which n is an integer having a value of from 4 to 29 and are mismatched analogs of complexes of polyribocytidylate (rCₙ) e.g., by including 2′-O-methyl ribosyl residues. These mismatched analogs of rIₙ.rCₙ, preferred ones of which are of the general formula rIₙ.r(C₁₂,U)n, are described by Carter and Ts'o in U.S. Patents 4,130,641 and 4,024,222. The dsRNA's described therein generally are suitable for use according to the present invention.

Specific examples of mismatched dsRNA for use in the invention include:-
poly (I)· poly (C₄,U)
poly (I)· poly (C₇,U)
poly (I)· poly (C₁₃,U)
poly (I)· poly (C₂₂,U)
poly (I)· poly (C₂₀,G)
poly (I)· poly (C₂₉,G) and
poly (I)· poly (Cₚ) 23 G>p
The amount of mismatched dsRNA administered is preferably sufficient to achieve a peak blood concentration of from 0.01 micrograms per milliliter of dsRNA up to 1000 micrograms per milliliter of dsRNA in the systemic blood circulation immediately following administration distal from the point of infusion.

I report herein three individuals with leukemia (CML), who demonstrated the novel RNA-ase L defect in association with uncontrolled growth of their malignant tumor cells and significant clinical deterioration (malaise, weight loss, inability to withstand multiple infections) with various associated virus infections including herpes zoster, CMV, EBV, herpes simplex, or hepatitis. Several patients also experienced chronic fungus infections of the mouth.

I both elucidated the novel biochemical derangement and devised a schedule of dsRNA administration, alone or in combination with a lymphokine, which effectively corrected the RNA-ase L abnormality and thereby led to dramatic clinical improvement characterized by significant reduction in the tumor burden and significant improvement in the overall antiviral and immunological defenses as evidenced by reduction in intercurrent viral and fungal infections.

Lymphokines include the interferons (alpha, beta, gamma), preferably interferon alpha, the interleukins, specifically interleukins (1, 2 or 3) and recombinant interleukin-2 (rIL-2), and tumor necrosis factor (TNF). Also included are lymphokine activated killer cells (LAK) formed in animals in response to exposure to a lymphokine.

When interferon (alpha) is used as the lymphokine, an amount of from 0.01 to 100,000 IRU per milliliter of the patient's body fluid is provided. When IL-2, preferably rIL-2, is the lymphokine, the amount administered lies within a range of about 10² IL-2 units per kg of the patient's body weight up to a value approaching unacceptable levels of toxicity in that patient, which may be as high as 10⁶ IL-2 units. However, most effective, toxic-reaction manageable values are in the range of from about 10³ to about 10⁴ IL-2 per kg of body weight.

When both agents, the dsRNA and the lymphokine, are administered as previously described they may be administered as a mixture, administered separately but simultaneously, or sequentially.

Administration of a dsRNA and a lymphokine "in combination" includes presentations in which both agents are administered together as a therapeutic mixture, and also procedures in which the two agents are administered separately but simultaneously, e.g., as through separate intravenous lines into the same individual. Administration "in combination" further includes the separate administration of one of the drugs in which one of the drugs is given first followed shortly by the second.

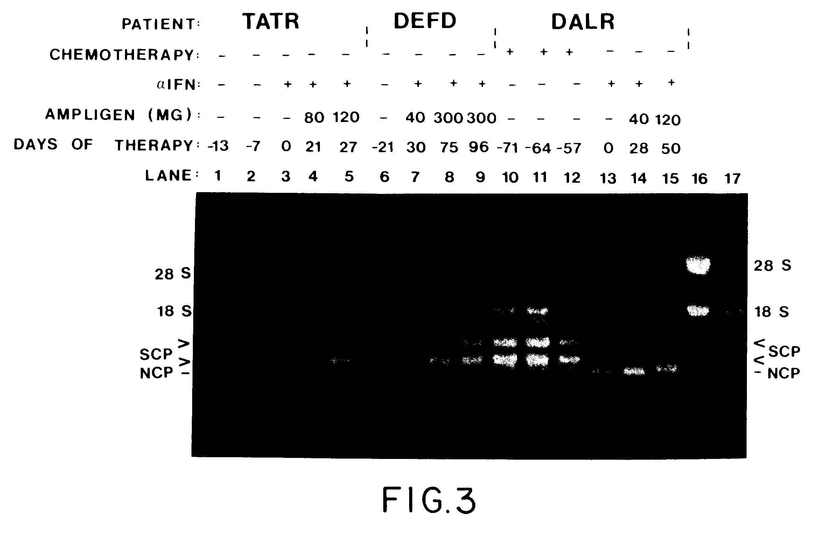

Figure 3 shows typical restoration of biochemical recovery in 3 patients (TATR, DEFD, DALR) first exposed to low dose lymphokine (alpha IFN in doses of 0.5 to 3.0 million IRU/4-7 times per week). Each patient weighed about 60 kilograms. Note that in each case the introduction of lymphokine alone was clearly insufficient to cause any detectable improvement in the RNA-ase L defect. Specifically, all patients showed only NCP when receiving only the lymphokine. Patient (DALR) had a brief period of response to conventional chemotherapy (hydroxyurea, etc.) during which his lymphocytes transient regained SCP activity; however, his disease relapsed with a conversion from SCP to NCP associated in time with clinical deterioration and he was placed on lymphokine with measurable clinical or biochemical improvements. In each instance in which dsRNA was then introduced, in conjunction with the lymphokine, dramatic clinical improvements and restoration of biochemical normalcy of RNA-ase L occurred apparently simultaneously.

Figure 3 shows the effectiveness of the mismatched dsRNA rIₙ.r(C₁₁₋₁₄,U)ₙ or AMPLIGEN® (a registered trademark of HEM Research, Rockville, Maryland, USA) given over a dosage range of 40 to 300 mg (applied intravenously twice weekly). I observed that I could achieve similar clinical effects by monitoring the enzyme (RNA-ase L) and administering mismatched dsRNA alone, but that the amount of dsRNA required was often 2 to 20 times greater and the onset of clinical response was significantly delayed.

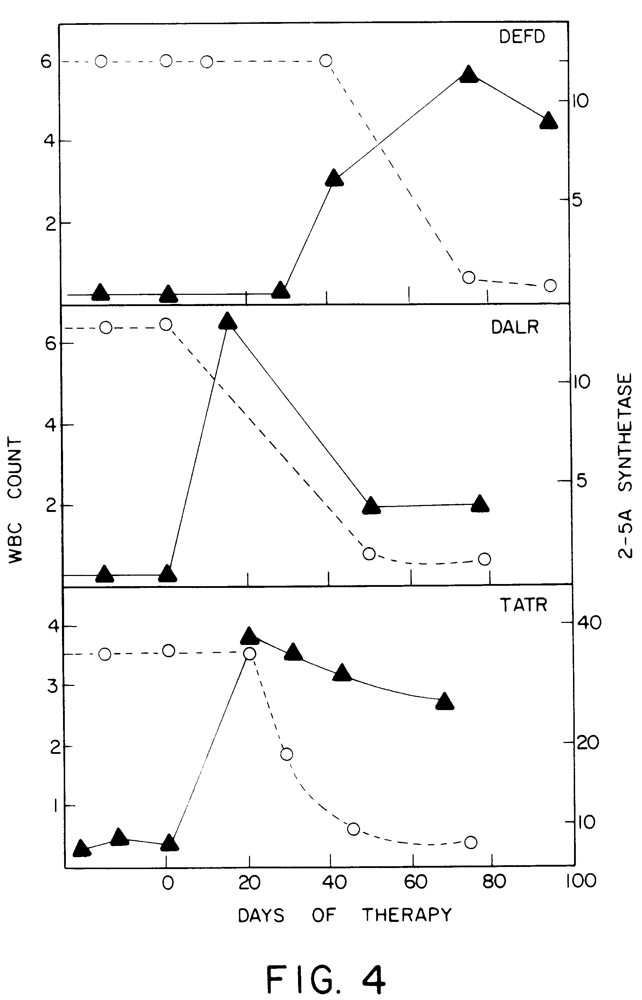

By combining the RNA-ase L assay and judiciously utilizing the mismatched RNA alone or in combination with lymphokines, I have been able to consistently improve overall clinical status (decreased viral and fungal infections) while reducing tumor burden significantly (as shown in Figure 4, discussed below). Importantly, it is now possible to effect these significant clinical changes on a cost/effective basis and also to minimize, if not eliminate, patient toxicity to these reagents. Thus, my invention is widely applicable to a variety of lymphokines as well as to a large family of double-stranded RNAs which might otherwise be of little to no use clinically unless this strategy were employed.

The graph of Figure 4 is illustrative of the dramatic biochemical and clinical effects achieved in the 3 patients with leukemia whose RNA-ase L profiles are reported in Figure 3. In Figure 4, the white blood count (cells per cubic milliliter, represented by the connected "O"s), and peripheral lymphocyte 2′-5′ A synthetase activities (represented by the connected deltas) are both plotted over time. To isolate the lymphocytes for 2′-5′ A synthetase measurement, approximately 1 x 10⁶ mononuclear cells in peripheral blood were first purified by the standard Ficol Hypaque technique. In all three instances, prior chemotherapy or prior lymphokine therapy had been of very limited utility, as evidenced by high white blood cell (WBC) counts, i.e., greater than 3,000 per cubic milliliter, and low 2′-5′ A synthetase, expressed as nanomoles per mg of cellular protein. However, mismatched dsRNA, added to cells which had first been "primed" with a low dose of lymphokine, showed concurrent reduction in the number of circulating tumor cells (WBC count), return to normalcy of lymphocyte RNA-ase L and improvement in overall antiviral state. The clinical effectiveness of such regimens is of unusually long duration and some of the patients may ultimately meet rigorous clinical criteria of "cure". I evaluated this possibility in relevant animal models in which I demonstrated not only tumor growth inhibition, but increased survival (p<0.001) by scheduling dsRNA treatment, alone or in combination with lymphokines, based on the relative level of recovery of RNA-ase L and resuscitation of the entire 2′-5′ A/synthetase/RNA-ase L pathway. I also noted that such treatments augmented natural immunosurveillance (NK cells, LAK cells, etc.), as well as being directly beneficial by reducing the number of viable tumor cells.

Additional, confirmatory studies indicated that these scheduled treatments, i.e., dsRNA alone or in combination with lymphokines, actually rendered the various tumor cells more sensitive to lysis even though the lymphokines alone generally did not effectively increase either tumor target cell sensitivity or dramatically enhance immune function.

## Claims

1. The use of a double-stranded RNA in the manufacture of a composition for the correction of a deficiency in RNase L, which deficiency manifests itself in the production of cleavage products other than the Specific Cleavage Products (SCPs) when the deficient RNase L is incubated with 18 S and 28 S rRNA, wherein correction of the deficiency is effective in the treatment of a disease.

2. The use of a double-stranded RNA according to claim 1 in which the double-stranded RNA is any double-helical RNA capable of serving as both a co-factor for intracellular 2'-5' A synthetase and generator of specific bioactive 2'-5' A oligomers which can activate RNA-ase L.

3. The use of a double-stranded RNA according to claim 1 in which a mismatched dsRNA is used, preferably rIₙ.r(c₁₁₋₁₄,U)ₙ.

4. The use of a double-stranded RNA according to claim 3 in which the mismatched dsRNA is administrated in an amount which results in a level of from 1 to 1,000 micrograms of the mismatched dsRNA per milliliter of the patient's body fluid.

5. The use of claim 1, optionally in combination with lymphokine therapy, wherein the disease is cancer, an immunological disorder or a viral infection.

6. The use of claim 5 wherein the disease is cancer and the tumour cells are resistant to treatment with interferon alone.

7. The use of claim 5 wherein the dsRNA is constructed from oligomer complexes, polymer complexes or both.

8. The use of claim 7 wherein the dsRNA is constructed of oligonucleotides hybridised with complimentary, single stranded polymeric RNA to form the dsRNA duplex.

9. The use of any one of claims 5 to 8 wherein the dsRNA contains regions of bond breakage and exhibits the favourable therapeutic ratio property of rIₙ.r(c₁₁₋₁₄,U)ₙ.

10. The use of claim 5 wherein a lymphokine is administered concurrently with the dsRNA.

## Patentansprüche

1. Verwendung einer doppel-strängigen RNA zur Herstellung einer Zusammensetzung zur Korrektur einer Defizienz in RNase L, welche Defizienz sich in der Herstellung von Spaltprodukten anders als die spezifischen Spaltprodukte (SSP) äußert, wenn die mangelhafte RNase L mit 18 S und 28 S rRNA inkubiert wird, wobei die Korrektur der Defizienz in der Behandlung einer Erkrankung wirksam ist.

2. Verwendung einer doppel-strängigen RNA nach Anspruch 1, wobei die doppel-strängige RNA irgendeine doppel-helikale RNA ist, die in der Lage ist, sowohl als Co-Faktor für eine intrazelluläre 2'-5' A Synthetase als auch als Generator von spezifischen bioaktiven 2'-5' A-Oligomeren zu dienen, die RNA-ase L aktivieren können.

3. Verwendung einer doppel-strängigen RNA nach Anspruch 1, wobei eine mismatched dsRNA, vorzugsweise rIₙ.r(C₁₁₋₁₄,U)ₙ, verwendet wird.

4. Verwendung einer doppel-strängigen RNA nach Anspruch 3, wobei die mismatched ds RNA in einer Menge verabreicht wird, die zu einem Level von 1 bis 1000 Microgramm der mismatched dsRNA pro ml der Patienten-Körperflüssigkeit führt.

5. Verwendung nach Anspruch 1, ggfs. in Kombination mit einer Lymphokin-Therapie, wobei die Erkrankung Krebs, eine immunologische Störung oder eine virale Infektion ist.

6. Verwendung nach Anspruch 5, wobei die Erkrankung Krebs ist und die Tumorzellen gegenüber einer Behandlung mit Interferon alleine resistent sind.

7. Verwendung nach Anspruch 5, wobei die dsRNA aus Oligomer-Komplexen, Polymer-Komplexen oder beiden konstruiert ist.

8. Verwendung nach Anspruch 7, wobei die dsRNA aus Oligonukleotiden konstruiert ist, die mit einer komplementären, einzelsträngigen polymeren RNA zur Ausbildung des dsRNA-Duplex hybridisierten.

9. Verwendung nach einem der Ansprüche 5 bis 8, wobei die dsRNA Bindungs-Bruchstellen-Bereiche enthält und die günstige Therapie-Verhältnis-Eigenschaft von rIₙ.r(C₁₁₋₁₄,U)ₙ aufweist.

10. Verwendung nach Anspruch 5, wobei ein Lymphokin gleichzeitig mit der dsRNA verabreicht wird.

## Revendications

1. Utilisation d'un ARN double-brin dans la fabrication d'une composition utile pour la correction d'une déficience en RNase L, déficience qui se manifeste elle-même par la production de produits de clivage autres que les Produits de Clivage Spécifiques (SCP) lorsque la RNase L déficiente est incubée avec L'ARNr 18S et 28S, dans laquelle la correction de la déficience est efficace dans le traitement d'une maladie.

2. Utilisation d'un ARN double-brin selon la revendication 1, dans laquelle l'ARN double-brin est un ARN double-hélice quelconque, capable de servir à la fois de cofacteur pour la 2'-5' A synthétase intracellulaire et de générateur d'oligomères 2'-5' A bioactifs spécifiques qui peuvent activer la RNAase L.

3. Utilisation d'un ARN double-brin selon la revendication 1, dans laquelle un ARNdb mésapparié est utilisé, de préférence rIₙ·r(c₁₁₋₁₄,U)ₙ.

4. Utilisation d'un ARN double-brin selon la revendication 3, dans laquelle l'ARNdb mésapparié est administré en une quantité qui se traduit par un taux de 1 à 1000 microgrammes de l'ARNdb mésapparié par millilitre de fluide corporel du patient.

5. Utilisation selon la revendication 1, en combinaison éventuellement avec une thérapie à la lymphokine, dans laquelle la maladie est un cancer, un trouble immunologique ou une infection virale.

6. Utilisation selon la revendication 5, dans laquelle la maladie est un cancer et les cellules tumorales sont résistantes au traitement avec un interféron seul.

7. Utilisation selon la revendication 5, dans laquelle l'ARNdb est construit à partir de complexes d'oligomères, de complexes de polymères, ou des deux à la fois.

8. Utilisation selon la revendication 7, dans laquelle l'ARNdb est composé d'oligonucléotides hybridés avec un ARN polymère simple-brin complémentaire, pour former l'ARNdb duplex.

9. Utilisation selon l'une quelconque des revendications 5 à 8, dans laquelle l'ARNdb contient des régions de cassure de liaison et manifeste la propriété de rapport thérapeutique favorable de rIₙ·r(c₁₁₋₁₄,U)ₙ.

10. Utilisation selon la revendication 5, dans laquelle une lymphokine est administrée simultanément à l'ARNdb.
